**Europäisches Patentamt**

**European Patent Office**

**Office européen des brevets**

(11) Veröffentlichungsnummer: **0 007 387**
**B1**

(12) **EUROPÄISCHE PATENTSCHRIFT**

(45) Veröffentlichungstag der Patentschrift:
**28.10.81**

(21) Anmeldenummer: **79101228.9**

(22) Anmeldetag: **24.04.79**

(51) Int. Cl.³: **C 07 C 17/42,** C 07 C 17/38

(54) **Neutralisationsverfahren für Chlorkohlenwasserstoffe.**

(30) Priorität: **31.07.78 DE 2833586**
**31.07.78 DE 2833548**

(43) Veröffentlichungstag der Anmeldung:
**06.02.80 Patentblatt 80/3**

(45) Bekanntmachung des Hinweises auf die Patenterteilung:
**28.10.81 Patentblatt 81/43**

(84) Benannte Vertragsstaaten:
**BE CH DE FR GB IT NL SE**

(56) Entgegenhaltungen:
**JAPANESE PATENTS REPORT, Part I-Chemical, Derwent Publications Ltd., Vol. 75, Nr. 51, No. J7-5037642**

**CHEMICAL ABSTRACTS, Vol. 81, Nr. 19, 11. November 1974, Nr. 119905 m Seite 473, Columbus, Ohio, U. S. A. DE-A-1 935 744**

(73) Patentinhaber: **DYNAMIT NOBEL AKTIENGESELLSCHAFT, Patentabteilung Postfach 1209, D-5210 Troisdorf, Bez. Köln (DE)**

(72) Erfinder: **Bieding, Robert, Drachenfelsstrasse 14, D-5463 Unkel (DE)**
Erfinder: **Nestler, Heinz, Dr., Rembrandtstrasse 73, D-5210 Troisdorf-Sieglar (DE)**

ACTORUM AG.

## Neutralisationsverfahren für Chlorkohlenwasserstoffe

Gegenstand der vorliegenden Erfindung ist ein Verfahren, Chlorkohlenwasserstoffe, insbesondere bereits verwendete Chlorkohlenwasserstoffe, zu neutralisieren bzw. eine Ansäuerung dieser Verbindungen zu verhindern. Das Verfahren eignet sich besonders gut zur Wiederaufbereitung von 1,1,1-Trichloräthan, das aus technischen Reinigungsanlagen zurückgewonnen wird. Die Zurückgewinnung erfolgt dabei aus der Abluft der Anlagen durch Adsorption an Aktivkohle und nachfolgender Desorption mittels Wasserdampf.

Chlorkohlenwasserstoffe sind bekannte Lösungsmittel, die im grossen Umfang zur Entfettung und/oder Reinigung von Metalloberflächen eingesetzt werden. Da in zunehmendem Masse solche Metalloberflächen gereinigt oder entfettet werden müssen, die oberflächlich Reste von Bohrölemulsionen oder von vorgeschalteten sauren Spülbädern enthalten, werden dadurch die zur Reinigung eingesetzten Chlorkohlenwasserstoffe angesäuert. Solche sauren Chlorkohlenwasserstoffe führen bei der Metallreinigung jedoch zu Korrosionserscheinungen an Reinigungsgut und Reinigungsanlagen. Der Säuerungsprozess kann dabei unter Umständen soweit fortschreiten, dass infolge Hydrolyse eine weitergehende Zersetzung des Lösungsmittels unter Bildung erheblicher Mengen an Chlorwasserstoff eintritt.

Diese starke Beanspruchung des Lösungsmittels kann nicht allein durch die an sich bekannten Stabilisatoren, die heute in jedem technisch eingesetzten Chlorkohlenwasserstoff vorhanden sind, ausgeglichen werden. Es werden deshalb in den Metallreinigungsanlagen die Chlorkohlenwasserstoffe in einer abgetrennten Anlage neutralisiert und anschliessend wieder der Reinigungsanlage zugeführt. Zur Neutralisation werden unter anderem wässrige Sodalösungen, Amine oder wässrige Aufschlämmung von Schlämmkreide oder gelöschtem Kalk eingesetzt. Auch die Lagerung von Marmorstücken oder sodabeladener Keramikkörper in dem Lösungsmittel wurde schon zur Neutralisation verwendet.

Diese bekannten Neutralisationsmittel bzw. Neutralisationsverfahren führen jedoch zu unbefriedigenden Ergebnissen, weil dabei Verbindungen entstehen, die teilweise in den Chlorkohlenwasserstoffen löslich sind und deren Wirkung dementsprechend herabsetzen, oder aber ihre Wirkung ist wegen ungenügender Porosität nicht ausreichend. Einige der bekannten Neutralisationsmittel wirken auch nicht schnell genug oder binden die Säure nicht über eine genügend lange Zeit.

Als Neutralisationsmittel werden z.B. in der JP-OS 69603/74 NaOH oder CaO genannt. Gemäss dieser Literaturstelle werden diese Neutralisationsmittel den Chlorkohlenwasserstoffen hinzugefügt und verbleiben in diesen in gelöster Form. Dadurch verunreinigen sie den Chlorkohlenwasserstoff und können auch Anlass zu unerwünschten Sekundärreaktionen geben, wie z.B. von Chloracetylen beim Einsatz von CaO.

Auch wenn solche bekannten Neutralisationsmittel auf Trägermaterialien aufgebracht sind, wie es in der JP-OS 37 642/75 beschrieben ist, treten die gleichen Nachteile ein: die dort genannten Neutralisationsmittel lösen sich in dem Chlorkohlenwasserstoff und verunreinigen diesen. Auch bedingt das Arbeiten mit Trägermaterialien einen zusätzlichen apparativen Aufwand, der unerwünscht ist.

Auch bei der Wiedergewinnung von Chlorkohlenwasserstoffen aus Absaugevorrichtungen in Metallreinigungsanlagen genügt diese Neutralisation, die im allgemeinen zusammen mit einer Nachstabilisation durchgeführt wird, nicht.

In diesen Absaugvorrichtungen wird das in der Abluft der Absaugung befindliche Lösemittel durch Adsorption an Aktivkohle und anschliessende Desorption durch Überleiten von Wasserdampf wiedergewonnen. Im Gegensatz zu Perchloräthylen und Trichloräthylen, die bei entsprechender Qualität nach der Rückgewinnung ausreichende Stabilität für den Wiedereinsatz zeigen, erfolgt bei stabilisiertem 1,1,1-Trichloräthan ein fast völliger Abbau der Stabilisatoren sowie eine Säuerung des Lösemittels durch Hydrolyse. Der Wiedereinsatz eines unbehandelten 1,1,1-Trichloräthan-Desorbats führt zwangsläufig zur Säuerung des gesamten Lösemittels der Reinigungsanlage.

Bei den bisher bekannten Verfahren zur Wiederverwendung des desorbierten 1,1,1-Trichloräthans erfolgt eine Entsäuerung und Nachstabilisierung durch Zugabe eines Gemischs, das mindestens aus einem der oben genannten, bekannten, flüssigen Neutralisationsmittel und einem Stabilisatorkonzentrat besteht.

Diese Methode weist eine Reihe von Nachteilen auf, die den Betriebsablauf stören und schliesslich sogar zu Schäden an der Anlage führen können. Einmal kann diese Behandlung nur chargenweise erfolgen, was dazu führt, dass die Lagerung einer bis Chargengrösse wachsenden Menge sauren Lösemittels notwendig ist. Dies führt im Laufe der Zeit zu Korrosionsschäden am Behältermaterial und zu einer fortschreitenden Beeinträchtigung des Trichloräthans.

Weiterhin lässt sich das Vorliegen eines bestimmten, wenn auch zunächst frei wählbaren Verhältnisses der Mengen Neutralisationsmittel und Stabilisatorkonzentrat im Zugabegemisch keine Anpassung an wechselnde Betriebsverhältnisse zu. So ist es möglich, dass bei der Koppelung der Zugabemenge des Gemisches an die aktuelle Azidität des Desorbats die sich dabei automatisch ergebende Zusatzmenge an Stabilisator nicht den optimalen Betriebsbedingungen entspricht. Umgekehrt kann bei optimaler Trichloräthanmenge die Konzentration des Neutralisationsmittels zu hoch oder zu niedrig sein.

Ausserdem können die bisher verwendeten Neutralisationsmittel in dem eingesetzten Gemisch zur Bildung flüssiger Neutralisationsprodukte führen, die sich im Lösemittel anreichern und dieses verunreinigen oder in seiner Wirkung beeinträchtigen.

Es bestand deshalb die Aufgabe, die in Metallreinigungsanlagen anfallenden Chlorkohlenwasserstoffe so zu behandeln, dass eine Säuerung nicht auftreten kann oder eine bereits vorhandene Säuerung so zu neutralisieren, dass keine schädlichen Nebenprodukte in den Lösungsmitteln auftreten und die Neutralisationswirkung schnell eintritt und dauerhaft bleibt.

In Erfüllung dieser Aufgabe wurde nun ein Verfahren zur Entsäuerung und Aufrechterhaltung der Säurefreiheit von Chlorkohlenwasserstoffen durch Behandeln mit Neutralisationsmitteln gefunden, das dadurch gekennzeichnet ist, dass man die Chlorkohlenwasserstoffe mit Magnesiumoxid in Kontakt bringt. Gegebenenfalls, z.B. bei der Neutralisation eines Desorbats, kann daran anschliessend eine Nachstabilisierung in an sich bekannter Weise erfolgen.

Es ist zwar auch schon bekannt, Magnesiumoxid zum Stabilisieren von paraffinischen Chlorkohlenwasserstoffen einzusetzen. Dort wird das Oxid jedoch stets gemeinsam mit Epoxidverbindungen eingesetzt, da es zusammen mit diesen einen synergistischen Effekt bei der Stabilisierung des Chlorparaffins ausübt. Es wird deshalb dort nur in Mengen von 0,000001 bis 1 Gew.-%, bezogen auf das Chlorparaffin, eingesetzt. Diese geringen Mengen können nicht neutralisierend wirken. Ausserdem muss dort die Magnesiumverbindung sich in dem Chlorparaffin lösen; dies ist im vorliegenden Fall nicht notwendig.

Bei dieser erfindungsgemässen Verfahrensweise treten die Nachteile der bisher bekannten Verfahren nicht auf. Es entstehen keine Nebenprodukte, die die Chlorkohlenwasserstoffe eventuell verunreinigen könnten, und die Wirkungsweise hält über mehrere Wochen an. Auch ist es möglich, stark saure Chlorkohlenwasserstoffe so zu neutralisieren, dass nach der Behandlung der Säuregehalt unter 1 ppm liegt.

Das einzusetzende Magnesiumoxid soll möglichst eine Korngrösse von 1 bis 100 µm, vorzugsweise von 2 bis 30 µm besitzen. Es kann auch mit Hilfe von geeigneten Bindemitteln zu Formkörpern verpresst und anschliessend ausgehärtet werden. Eine solche Einsatzform eignet sich besonders gut und zeigt die gleiche Wirkung wie die ungebundene, pulverige Form.

Als Bindemittel zur Herstellung von $M_g$O-Formkörpern eignen sich hauptsächlich Orthokieselsäureester der $C_1$-bis $C_4$-Alkohole, die entsprechenden oligomeren Kieselsäureester und die Hydrolysate dieser Verbindungen; ferner Titanate, Magnesiumchlorid, Alkalisilikate oder Alkalicarbonate. In allen Fällen wird pulveriges Magnesiumoxid mit dem Bindemittel vermischt, wobei die genannten Salze in Form konzentrierter Lösungen und die Kieselsäureester bzw. Titanate unter Zusatz der für die Hydrolyse dieser Verbindungen nötigen Mengen Wasser eingesetzt werden. Es soll möglichst eine zähviskose Masse vorliegen, die anschliessend geformt und daraufhin zum Aushärten getrocknet wird. Zum Trocknen genügen Temperaturen bis zu 150°C. Man erhält dann harte und abriebfeste Formkörper.

Die Grösse der Formkörper spielt keine Rolle für deren erfindungsgemässe Wirkungsweise. Es sind sowohl Rundkörper mit z.B. 5 cm Durchmesser und 5 cm Höhe als auch Körper in Ziegelsteinformat einsetzbar. Die Körper weisen dank ihrer Porosität ein gutes Eindringvermögen für die Chlorkohlenwasserstoffe auf; ein bevorzugtes Nachlassen der Absorbierfähigkeit für Säuren an der Körperaussenseite konnte nicht festgestellt werden.

Bevorzugte MgO-Formkörper sind solche, die mit Hilfe von Alkalicarbonaten hergestellt werden.

Die je nach Bedarf passend geformten Körper werden an geeignet erscheinenden Stellen in der Anlage untergebracht; die Chlorkohlenwasserstoffe werden mit dem MgO – in Pulverform oder in gebundener Form als Formkörper – auf beliebige Weise in Kontakt gebracht. Es genügt ein Überleiten der Lösungsmittel über das Pulver; am geeignetsten verfährt man jedoch so, dass man das Pulver oder die Körper an die Stellen plaziert, an denen die Chlorkohlenwasserstoffe über einen längeren Zeitraum verbleiben. Die Behandlung mit MgO wird gegebenenfalls räumlich von der Nachstabilisierung getrennt und möglichst nahe an den Ort der Entstehung der sauren Lösemittel bzw. Lösemittel/Wassergemische gelegt, wie z.B. in den Metallreinigungsanlagen im Arbeitsbecken einer Kalttauchanlage. Diese Plazierungsweise hat den Vorteil, dass überhaupt keine Säuerung des Chlorkohlenwasserstoffes auftreten kann, so dass Korrosionserscheinungen selbst im Arbeitsbecken nicht auftreten können.

Wenn ein Desorbat von 1,1,1-Trichloräthan erfindungsgemäss behandelt werden soll, dann soll diese Behandlung möglichst nahe benachbart zu der Adsorptionszone, entweder im Bereich der Kondensation oder im Abfluss aus dem Kondensator oder im Wasserabscheider und/oder im Desorbat-Sammelgefäss erfolgen. Bei den heute in Gebrauch befindlichen Anlagen wird es der Konstruktion wegen in der Regel nur möglich sein, die Formkörper im Wasserabscheider und im Desorbat-Sammelgefäss zu plazieren; bei Neukonstruktionen können auch an den anderen, oben bezeichneten Stellen geeignet geformte, bequem zugängliche, mit Deckel verschliessbare Behältnisse zur Aufnahme von Formkörpern vorgesehen werden. Auf diese Weise wird erreicht, dass es zu keiner Lagerung grösserer Mengen sauren Desorbats, wie oben beschrieben, kommen kann.

Weiterhin ist es auch möglich, die Behandlung mit dem MgO in dem Vorrätsbecken für den Rücklauf einer Lösemittelrückgewinnungsanlage durchzuführen.

Wenn Magnesiumoxid-Formkörper eingesetzt werden, dann sind diese in der Lage, langfristig Säurespuren aus dem Desorbat aufzunehmen. Die Körper verfestigen sich während des Gebrauchs, ohne dabei kurzfristig merklich an Wirksamkeit einzubüssen. Sind die Formkörper in ihrer Säureaufnahmekapazität erschöpft, was sich z.B. durch Titration einer Probe daraus leicht ermitteln lässt, werden sie durch frische ersetzt.

Die bevorzugte Arbeitsweise mit Formkörpern aus MgO bringt eine Reihe entscheidender Vorteile mit sich: Die Handhabung ist einfach und führt zu keinerlei Umweltbelastungen. Das Neutralisationsmittel wird stets im Überschuss stationär angeboten, ohne dass dies das Lösemittel in irgendeiner Weise negativ beeinflusst. Die Säurespuren werden ihm so frühzeitig wieder entzogen, dass die bei der nicht erfindungsgemässen Fahrweise solcher Anlagen recht grosse Gefahr der Korrosion im Bereich der Kondensator-Sammelbehälter stark eingeschränkt wird. In einem feuchten, weitgehend stabilisatorfreien Desorbat werden dann die üblicherweise durch Säure katalysierten Zersetzungsvorgänge mangels Katalysator nicht mehr angeregt.

Im Falle, dass ein teilweise entstabilisiertes Desorbat erfindungsgemäss behandelt wird, gestaltet sich eine anschliessende Nachstabilisierung wesentlich übersichtlicher und problemloser als bei der Fahrweise nach dem Stand der Technik. Bei diesem Schritt wird das Lösemittel mit der Menge an Stabilisatoren versetzt, die während der gesamten vorangegangenen Prozesse verlorengegangen ist. Dies erfolgt entweder durch frisches, stabilisiertes 1,1,1-Trichloräthan oder durch ein solches 1,1,1-Trichloräthan, das die gewünschten Stabilisatoren konzentriert enthält. Auch die direkte Zugabe der einzelnen Stabilisatoren ist möglich.

Bei den bisher genannten Verfahren dagegen musste man in diesen Fällen zunächst die vorhandene Säuremenge, die bei der Menge der zuzusetzenden Nachstabilisierlösung zu berücksichtigen ist, analytisch feststellen, da man erfahrungsgemäss nicht mit einem ständig gleichbleibenden Zustand des anfallenden Lösemittels rechnen konnte. Die Nachstabilisierlösung sollte dann die von Fabrikat zu Fabrikat variierende Zusammensetzung des Stabilisators der entsprechenden 1,1,1-Trichloräthantype wiederherstellen und sie enthielt daher ein ähnliches Gemisch an Stabilisatorkomponenten, zuzüglich einer neutralisierenden Komponente, sowie das Lösemittel. Übliche Stabilisatorkomponenten, die auch anschliessend an die erfindungsgemässe Behandlung eingesetzt werden, sind z.B. entnommen aus den Gruppen der tert.-Alkohole, Epoxide, Ringäther, Amine. Je nach Art und Menge der im Desorbat vorhandenen Säuren hatten sich bei den bisher bekannten Verfahren entweder eine oder mehrere Stabilisatorkomponenten, mehr oder weniger anteilig, im Zuge der Neutralisationsreaktion umgesetzt, so dass nur der Rest als wirksamer Stabilisator erhalten blieb. Es war dadurch schwierig, den genauen Gehalt an Stabilisatoren im zurückgewonnenen Lösemittel und deren Zusammensetzung vorherzusehen.

Diese Nachteile entfallen bei dem erfindungsgemässen Verfahren: Wenn ein nach dem vorliegenden Verfahren erhaltenes neutrales Desorbat nachstabilisiert werden soll, so ist die Zusammensetzung der Nachstabilisierlösung nur vom analytisch erfassbaren Grad des Abbaus der ursprünglich vorhandenen Stabilisatoren während der Adsorption/Desorption abhängig. Das dem Bedarf genau angepasste, zugesetzte Stabilisatorgemisch steht ohne Beeinträchtigung durch Nebenreaktionen voll zur Verfügung und man kann bei diesem Vorgehen die notwendige Menge an zu ergänzenden Stabilisatoren in viel engeren Grenzen festlegen, ohne ein Risiko einzugehen.

Ein weiterer technischer Aspekt dieser Konsequenz liegt darin, dass bei den für eine ausreichend wirksame Stabilisierung von Trichloräthan ohnehin mit üblicherweise 4 bis 6% an Zusätzen notwendigen grossen Mengen an Fremdstoffen jeder zusätzliche Überschuss vermieden werden kann, der dann in einem Kreislaufprozess seinerseits bei der Reaktion auf der Aktivkohle zu einem vermehrten Anfall von teils sauren Zersetzungsprodukten führen würde.

Darüber hinaus kann man bei eingefahrenen, gut gewarteten Anlagen nach einer erfindungsgemässen Behandlung mit MgO auf ein gesondertes Zugeben von Nachstabilisierlösung in das Desorbat ganz verzichten, wenn die Trichloräthan-Frischware die Fehlmenge an Stabilisatoren im Desorbat mit enthält und Frischware und Desorbat der Anlage in etwa gleichbleibendem Mengenverhältnis zugeführt werden.

Zu den Chlorkohlenwasserstoffen, die erfindungsgemäss behandelt werden können, zählen neben 1,1,1-Trichloräthan hauptsächlich Trichloräthylen und Perchloräthylen sowie andere chlorierte Alkane mit 1 bis 3 Kohlenstoffatomen. Diese, überwiegend als Lösungsmittel einsetzbaren Verbindungen können für die erfindungsgemässe Behandlung sowohl in stabilisierter Form vorliegen, wie es allgemein üblich ist oder auch in unstabilisierter Form, wenn sich dies in besonderen Fällen, wie z.B. bei der oben geschilderten Neutralisation eines Desorbats, ergibt.

In wirtschaftlicher Hinsicht erbringt das erfindungsgemässe Vorgehen Vorteile durch geringere Korrosionsgefahr in der Anlage, durch weniger aufwendige Analytik, durch grössere Sicherheit beim Arbeiten mit geringerer Störanfälligkeit, durch Wegfall der neutralisierenden Komponente in der Nachstabilisierlösung, die durch Magnesiumoxid-Formkörper ersetzt wird, und schliesslich durch eine Einsparung an Stabilisatoren, da jeder Überschuss daran vermieden werden kann.

Beispiel 1

In einem 2 l Rundkolben mit aufgesetztem Soxhlet wurden 1,5 l stabilisiertes, handelsübliches 1,1,1-Trichloräthan mit 1,5 Vol.-% Wasser versetzt und insgesamt 28 Tage am Rückfluss gekocht. Zur Verhinderung der Säuerung wurde 50 g pulverförmiges MgO einer mittleren Korngrös-

se von 3 μm in einer Extraktionshülse im Soxhlet plaziert.

In Abständen von jeweils 7 Tagen wurde das Lösungsmittel auf einen eventuellen Gehalt an freier Säure wie folgt untersucht: 50 ml Lösungsmittel wurden in einen mit Schliffstopfen versehenen Erlenmeyer-Kolben mit 25 ml neutral gestellter wässriger Bromthymolblaulösung als Indikator versetzt und unter Schütteln mit 0,01 n - NaOH versetzt, bis Grünfärbung eintrat. Bis zu einer Versuchsdauer von 28 Tagen trat Grünfärbung sofort ein, so dass praktisch keine freie Säure im Lösungsmittel vorhanden war.

Der Versuch wurde weiterhin auf drei Monate ausgedehnt. Auch in diesem Zeitraum zeigte das Lösungsmittel neutrale Reaktion.

Zum Vergleich wurde der Versuch in gleicher Weise ohne Plazierung von MgO in den Soxhlet wiederholt. Bereits nach einer Versuchsdauer von 7 Tagen hatte das Lösungsmittel einen Gehalt von 5 ppm HCl, der nach Ablauf von 28 Tagen auf 120 ppm angestiegen war.

Beispiel 2

In der im Beispiel 1 beschriebenen Apparatur wurden 1,5 l handelsübliches, stabilisiertes 1,1,1-Trichloräthan mit 1,5 Vol.-% Wasser versetzt und am Rückfluss während 10 Tagen gekocht. Nach dieser Zeit waren im Lösungsmittel 7,75 ppm HCl nachweisbar.

Daraufhin wurde in den Soxhlet 50 g Magnesiumoxid in einer Extraktionshülse eingebracht und das mit Wasser versetzte, chlorwasserstoffhaltige 1,1,1-Trichloräthan weitergekocht. Die gemäss Beispiel 1 im Laufe von jeweils 48 Stunden durchgeführten Analysen ergaben, dass die vorhandene Säure weitgehend abgebaut wurde und eine weitere Säurebildung zurückgedrängt wurde. Nach weiteren 4 Tagen lag der Säurewert unter 1 ppm und hielt sich auf diesem Niveau bis zum Abbruch des Versuchs nach insgesamt 21 Tagen.

Beispiel 3

In der in Beispiel 1 beschriebenen Apparatur wurden 1,5 l Per- bzw. Trichloräthylen mit 110% der zur Säuerung der alkalisch stabilisierten Lösungsmittelmengen notwendigen Menge wässriger Salzsäure versetzt und 24 Stunden am Rückfluss gekocht. Im Gegensatz zu Vergleichsversuchen ohne MgO-Zusatz, deren Lösungsmittel pH-Werte von ca 5,5 aufwiesen, zeigten Versuche mit MgO-Zusatz deutliche alkalische Reaktion der Lösungsmittel.

Beispiel 4

Zur Herstellung von Formkörpern wurden 500 g Magnesiumoxid mit 85 ml konzentrierter Sodalösung angeteigt und in eine Form gestampft. Nach Ausformen erfolgte 8stündige Trocknung bei 120°C. Der erhaltene Formkörper zeigte harte und abriebfeste Konsistenz.

In einer mit handelsüblichem, stabilisiertem 1,1,1-Trichloräthan gefüllten Metallreinigungsanlage (ca. 800 l) trat durch Hydrolyse in Folge plötzlichen Wassereintritts Säuerung des Lösungsmittels auf. Der ermittelte Säuregehalt betrug 8,5 ppm HCl. Es wurden 750 g der oben genannten MgO-Formkörper in den Wasserabscheider der Anlage gelegt. Dadurch wurde die weitere Säuerung des Lösungsmittels verhindert und die vorhandene Säure neutralisiert. 20 Stunden nach Plazierung der Formkörper konnte in der Anlage keine Säure mehr festgestellt werden.

Beispiel 5

Durch Plazierung von 450 g MgO-Formkörpern gemäss Beispiel 4 im Wasserabscheider einer Dampfentfettungsanlage (Lösungsmittelvolumen ca. 200 l) wurden Hydrolyse und Säuerung des stabilisierten 1,1,1-Trichloräthans wirksam verhindert, obwohl durch das Reinigungsgut aus einem vorgeschalteten Spülbecken kontinuierlich leicht saures Wasser in die Anlage eingeschleppt wurde.

Beispiel 6

In einer handelsüblichen 3-Kammer-Reinigungsanlage mit Adsorptionsanlage (2 mit Aktivkohle gefüllte Absorber) zur Rückgewinnung des 1,1,1-Trichloräthans aus der Abluft der Anlagenabsaugung wurde dieses durch Überleiten von Wasserdampf aus der mit 1,1,1-Trichloräthan beladenen Aktivkohle desorbiert. Das dabei anfallende Lösungsmittel-Wasserdampf-Gemisch wurde nach der Kondensation in einen 30 l fassenden Wasserabscheider geleitet und nach Abtrennung des Wassers in einem Auffangbehälter (Volumen 300 l) gesammelt. Der chargenweise Desorbatanfall betrug ca. 10 l/90 min. Daraus resultierte eine mittlere Verweilzeit des Lösemittels im Wasserabscheider von ca. 4½ Stunden.

Zur Neutralisation des sauer anfallenden 1,1,1-Trichloräthan-Desorbates (40-50 ppm HCl) wurden etwa 8,5 kg Magnesiumoxid-Formkörper (Zylinder mit 5 cm Durchmesser und Höhe) auf den Boden des Wasserabscheiders gelegt. Im, den Wasserabscheider verlassenden, Lösemittel konnte danach keine freie Säure mehr nachgewiesen werden.

Die MgO-Formkörper wurden folgendermassen hergestellt: 10 kg Magnesiumoxid werden mit 1700 ml konzentrierter Sodalösung angeteigt und in Teilmengen in entsprechende Formen gestampft. Nach dem Ausformen erfolgte 8stündige Trocknung bei 120°C. Die erhaltenen Formkörper hatten harte und abriebfeste Konsistenz.

Der Säuregehalt des Lösemittels wurde wie folgt ermittelt: 50 ml des Lösemittels wurden in einem mit Schliffstopfen versehenen Erlenmeyer-Kolben mit 25 ml neutral gestellter wässriger Bromthymolblaulösung (als Indikator) versetzt und unter Schütteln mit 0,01 n NaOH bis zum Farbumschlag nach grün titriert.

Zur Nachstabilisierung des Lösemittels wurde ein Konzentrat der im Ausgangsprodukt enthaltenen Stabilisatoren in 1,1,1-Trichloräthan verwendet. Die Konzentrationen der Nachstabilisatorlösung wurde so gewählt, dass bei 3%iger Zugabe zum 1,1,1-Trichloräthan-Desorbat ein Stabilisatoranteil von mindestens 80%, bezogen auf die Ausgangsstabilisierung, erreicht wurde.

**Beispiel 7**

Nach Einsatz von Mecloran D* (ein hochreines, inhibiertes 1,1,1-Trichloräthan für die Dampfentfettung) in der in Beispiel 6 beschriebenen Anlage wurde das neutralisierte und nachstabilisierte 1,1,1-Trichloräthan-Desorbat folgender Stabilitätsprüfung unterzogen (in Anlehnung an den Test der Bundesanstalt für Materialprüfung (BAM), Berlin, zur Untersuchung der chemischen Beständigkeit von inhibiertem 1,1,1-Trichloräthan in Gegenwart von Aluminium).

100 ml aufbereitetes Desorbat wurden mit 10 ml Toluol gemischt und mit 18 g Aluminiumflitter und 0,7 g $AlCl_3$ (wasserfrei) 18 Stunden am Rückfluss gekocht.

Die bei Verwendung von nicht nachbehandeltem 1,1,1-Trichloräthan zu beobachtende spontane Zersetzungsreaktion (Dehydrochlorierung und Polykondensation) des Lösemittels trat beim aufbereiteten Desorbat nicht auf. Das Reaktionsgemisch zeigte nach Beendigung des Tests lediglich eine leicht graugelbe Verfärbung.

**Beispiel 8**

Nach 6monatigem Einsatz von Mecloran D in der in Beispiel 6 beschriebenen Anlage, wobei insgesamt etwa 4500 kg 1,1,1-Trichloräthan-Desorbat mittels der Magnesium-Formkörper, die zwischenzeitlich nicht ausgewechselt wurden, im Wasserabscheider der Adsorptionsanlage neutralisiert wurden, zeigte das Desorbat einen nur geringen Gehalt an freier Säure ($< 2$ ppm). Nach Ergänzung der Stabilisatoren mit einem gemäss Beispiel 1 bereiteten Stabilisatorkonzentrat bestand das Lösemittel den in Beispiel 2 beschriebenen Test ohne Auftreten einer Zersetzungsreaktion.

**Patentansprüche**

1. Verfahren zur Entsäuerung und Aufrechterhaltung der Säurefreiheit von Chlorkohlenwasserstoffen durch Behandeln mit Neutralisationsmitteln, dadurch gekennzeichnet, dass man die Chlorkohlenwasserstoffe in Kontakt mit Magnesiumoxid bringt.

2. Verfahren gemäss Anspruch 1, dadurch gekennzeichnet, dass das Magnesiumoxid eine Korngrösse von 2 bis 30 µm besitzt.

3. Verfahren gemäss Ansprüche 1 oder 2, dadurch gekennzeichnet, dass ein Magnesiumoxid eingesetzt wird, das mit Hilfe eines Bindemittels aus der Gruppe Kieselsäureester, organische Titanate, Magnesiumchlorid, Alkalisilikate, Alkali- und Erdalkalicarbonate zu Formkörpern verformt werden.

4. Verfahren gemäss Anspruch 3, dadurch gekennzeichnet, dass ein Magnesiumoxid eingesetzt wird, das mit Hilfe von gesättigter wässriger Sodalösung zu Formkörpern verformt ist.

5. Verfahren gemäss Anspruch 1, dadurch gekennzeichnet, dass ein stabilisierter Chlorkohlenwasserstoff eingesetzt wird.

6. Verfahren gemäss einem der Ansprüche 1 bis 5, dadurch gekennzeichnet, dass man als Chlorkohlenwasserstoff 1,1,1-Trichloräthan einsetzt, das bereits zur Entfettung der Reinigung von Metallen eingesetzt war.

7. Verfahren gemäss einem der Ansprüche 1 bis 6, dadurch gekennzeichnet, dass ein durch Adsorption an Aktivkohle aus der Abluft technischer Anlagen und nachfolgende Desorption mittels Wasserdampf zurückgewonnenes 1,1,1-Trichloräthan, eingesetzt wird.

8. Verfahren gemäss Anspruch 7, dadurch gekennzeichnet, dass die Behandlung mit MgO im räumlichen Anschluss an den Ort der Desorption erfolgt.

**Claims**

1. Process for de-acidifying chlorinated hydrocarbons and keeping them acid free by treatment with neutralising agents, characterised in that the chlorinated hydrocarbons are brought into contact with magnesium oxide.

2. Process according to claim 1, characterised in that the magnesium oxide has a grain size of 2 to 30 µm.

3. Process according to claims 1 or 2, characterised in that a magnesium oxide is used which has been shaped to form moulded articles with the aid of a bonding agent selected from the group consisting of silicic acid esters, organic titanates, magnesium chloride, alkali silicate, alkali- and alkaline earth carbonates.

4. Process according to claim 3, characterised in that a magnesium oxide is used which is shaped to form moulded articles with the aid of saturated aqueous soda solution.

5. Process according to claim 1, characterised in that a stabilised chlorinated hydrocarbon is used.

6. Process according to one of claims 1 to 5, characterised in that there is used as chlorinated hydrocarbon 1,1,1-trichloroethane which has been used for the degreasing and purification of metals.

7. Process according to one of claims 1 to 6, characterised in that a 1,1,1-trichloro ethane is used which is recovered from the waste air of technical installations by adsorption on active carbon and subsequent desorption by means of water vapour.

8. Process according to claim 7, characterised in that the treatment with MgO is carried out in the vicinity of the location of desorption.

**Revendications**

1. Procédé pour désacidifier et maintenir des hydrocarbures chlorés exempts d'acide par un traitement à l'aide d'agents de neutralisation, caractérisé en ce que l'on met les hydrocarbures chlorés en contact avec de l'oxyde de magnésium.

2. Procédé selon la revendication 1, caractérisé en ce que l'oxyde de magnésium possède une granulométrie de 2 à 30 μm.

3. Procédé selon l'une des revendications 1 ou 2, caractérisé en ce que l'on met en œuvre un oxyde de magnésium qui a été façonné en des corps moulés au moyen d'un liant choisi dans le groupe des esters d'acide silicique, des titanates organiques, du chlorure de magnésium, des silicates alcalins, des carbonates alcalins et des carbonates alcalino-terreux.

4. Procédé selon la revendication 3, caractérisé en ce que l'on met en œuvre un oxyde de magnésium qui est transformé en des corps moulés à l'aide d'une solution aqueuse saturée de carbonate de sodium.

5. Procédé selon la revendication 1, caractérisé en ce qu'on utilise un hydrocarbure chloré stabilisé.

6. Procédé selon l'une quelconque des revendications 1 à 5, caractérisé en ce qu'on utilise comme hydrocarbure chloré du 1,1,1-trichloroéthane qui avait déjà été mis en œuvre pour le dégraissage et le nettoyage de métaux.

7. Procédé selon l'une quelconque des revendications 1 à 6, caractérisé en ce que l'on utilise un 1,1,1-trichloroéthane récupéré par adsorption sur du charbon actif à partir des gaz d'échappement d'installations industrielles et désorption ultérieure au moyen de vapeur d'eau.

8. Procédé selon la revendication 7, caractérisé en ce que le traitement à l'aide de MgO s'effectue dans un espace relié à l'endroit de la désorption.